# EUROPÄISCHE PATENTANMELDUNG

(11) **EP 0 876 799 A2**
(43) Veröffentlichungstag der Anmeldung: **11.11.1998**
(21) Anmeldenummer: 98107744.9
(22) Anmeldetag: 28.04.1998
(51) Int. Cl.: A61B 19/02

(54) **Medizinisch-technischer Systemarbeitsplatz**

(30) Priorität: 07.05.1997 DE 19719369; 31.03.1998 DE 19814367
(71) Anmelder: SIEMENS AKTIENGESELLSCHAFT, 80333 München (DE)
(72) Erfinder: Rattner, Manfred, 91091 Grossenseebach (DE); Malter, Bernd, 91090 Effeltrich (DE); Reichert, Thomas, 91056 Erlangen (DE); Helmreich, Gerhard, 91090 Effeltrich (DE)

(57) **Zusammenfassung**

Medizinisch-technischer Systemarbeitsplatz für die offene oder minimal invasive Chirurgie, aufweisend eine Ablageschale (11) und mindestens eine Anschlußeinheit (13) für Instrumente (22 - 26) medizinisch-technischer Geräte (G1 - G6), mindestens ein räumlich von der Anschlußeinheit (13) getrenntes Gerätecenter (3) zur Aufnahme der medizinisch-technischen Geräte (G1 - G6) und mindestens eine Verbindungseinheit (4), welche die Anschlußeinheit (13) und das Gerätecenter (3) miteinander verbindet.

## Beschreibung

Die Erfindung betrifft einen medizinisch-technischen Arbeitsplatz aufweisend Instrumente medizinisch-technischer Geräte, einen Geräteschrank zur Aufnahme der medizinisch-technischen Geräte und eine Verbindungseinheit, welche die Instrumente und den Geräteschrank miteinander verbindet.

In der offenen oder minimal invasiven Chirurgie kommen bei Operationen medizinisch-technische Geräte unterschiedlichster Funktionen gleichzeitig zum Einsatz. Die Geräte sind dabei in der Regel als eigenständige Einheiten ausgeführt und relative nahe um einen einen Patienten aufnehmenden Patientenlagerungstisch mehr oder weniger systematisch angeordnet. Als nachteilig erweist sich dabei, daß die Geräte einen nicht unerheblichen Platzbedarf im Bereich um den Patientenlagerungstisch haben, der einem aus Ärzten, Hilfs- und Bedienpersonal für die Geräte bestehenden Operationsteam nicht mehr zur Verfügung steht, so daß häufig das Arbeiten des Operationsteams auf engstem Raum notwendig ist.

Ein weiterer Nachteil der bekannten Arbeitsplätze liegt darin, daß allein für die Bedienung der Geräte mehrere Bedienpersonen notwendig sind, womit wirtschaftliche Nachteile verbunden sind.

Der Erfindung liegt daher die Aufgabe zugrunde, einen medizinisch-technischen Arbeitsplatz derart auszuführen, daß alle für einen offenen oder minimal invasiven chirurgischen Eingriff notwendigen medizinisch-technischen Geräte verfügbar sind und der Raum um den Patientenlagerungstisch dennoch gut zugänglich ist.

Nach der Erfindung wird diese Aufgabe gelöst durch einen medizinisch-technischen Systemarbeitsplatz für die offene oder minimal invasive Chirurgie aufweisend eine Ablageschale und mindestens eine Anschlußeinheit für Instrumente medizinisch-technischer Geräte, mindestens ein räumlich von der Anschlußeinheit getrenntes Gerätecenter zur Aufnahme der medizinisch-technischen Geräte und mindestens eine Verbindungseinheit, welche die Anschlußeinheit und das Gerätecenter miteinander verbindet. Die Ablageschale nimmt dabei alle für einen chirurgischen Eingriff notwendigen Instrumente der medizinisch-technischen Geräte auf, so daß sie für den Chirurgen auf einer einzigen Einheit zentral griffbereit sind. Die Anschlußeinheit ist dabei in der Regel unweit der Ablageschale angeordnet, um die Anschlußleitungen zwischen den Instrumenten und der Anschlußeinheit möglichst kurz zu halten. Da die einzelnen Geräte in einem räumlich von der Anschlußeinheit getrennten Gerätecenter angeordnet sind, welche über die Verbindungseinheit mit der Anschlußeinheit verbunden sind, wird der Raum um einen Patientenlagerungstisch des Systemarbeitsplatzes nicht mehr von einzelnen Geräten blockiert, so daß der Patientenlagerungstisch gut zugänglich ist. Unter einer räumlichen Trennung wird dabei verstanden, daß das Gerätecenter mindestens 1,5 Meter von dem Patientenlagerungstisch bzw. der Anschlußeinheit entfernt angeordnet ist, wobei das Gerätecenter sogar außerhalb des Operationssaales angeordnet sein kann. Ein weiterer Vorteil der Erfindung liegt darin, daß alle Geräte von einer einzigen Person zentral von dem Gerätecenter aus bedient werden können, wodurch Bedienpersonal eingespart werden kann. Dies wirkt sich vor allem wirtschaftlich positiv aus.

Ein medizinisch-technischer Arbeitsplatz der eingangs genannten Art ist beispielsweise aus der DE 92 18 373 U1 bekannt. Der Geräteschrank des Arbeitsplatzes ist jedoch unmittelbar neben dem Patientenlagerungstisch plaziert, so daß der Patientenlagerungstisch nicht frei zugänglich ist. Der Arbeitsplatz weist zudem keine Ablageschale zum zentralen Bereithalten der Instrumente auf.

Gemäß einer Variante der Erfindung sind die Ablageschale und die Anschlußeinheit an einer vorzugsweise beweglich ausgeführten Darreichungseinheit angeordnet. Auf diese Weise können die Ablageschale mit den Instrumenten der medizinisch-technischen Geräte und die Anschlußeinheit, welche über die Anschlußleitungen der Instrumente miteinander verbunden sind, gemeinsam, beispielsweise relativ zu einem Patientenlagerungstisch des Systemarbeitsplatzes, verstellt werden.

Eine besonders bevorzugte Ausführungsform der Erfindung sieht vor, daß die Ablageschale mindestens eine Bedieneinrichtung aufweist, welche über die Verbindungseinheit mit mindestens einem Steuerrechner des Systemarbeitsplatzes verbunden ist. Mit dem Steuerrechner sind auch die medizinisch-technischen Geräte verbunden. Der Steuerrechner wird dabei vorzugsweise mit einer menügesteuerten Software betrieben, welche es gestattet, alle an dem Steuerrechner angeschlossenen Geräte zentral von dem Steuerrechner aus zu bedienen. Auf diese Weise kann auch der Chirurg über die an den Steuerrechner angeschlossene Bedieneinrichtung, welche beispielsweise ein Joystick, ein Fußschalter oder eine Empfangseinheit zur Voice-Control sein kann, alle Geräte zentral von der Ablageschale aus bedienen, wobei in diesem Fall ein an den Steuerrechner angeschlossenes Anzeigegerät vorgesehen ist, welches vorzugsweise ein Monitor ist, welcher im Blickfeld des Chirurgen angeordnet ist. Indem Bedienaufgaben von dem Chirurgen selbst vorgenommen werden, gestattet es diese Ausführungsform der Erfindung, weiteres Bedienpersonal einzusparen, wodurch die Wirtschaftlichkeit des erfindungsgemäßen Systemarbeitsplatzes nochmals erhöht wird.

Eine Variante der Erfindung sieht vor, daß die medizinisch-technischen Geräte, die Bedieneinrichtung und der Steuerrechner über einen Kommunikationsbus miteinander verbunden sind. Der Kommunikationsbus stellt eine technisch besonders vorteilhafte Verbindung der einzelnen Geräte, der Bedieneinrichtung und des Steuerrechners miteinander dar, da er sich in vorteilhafter Weise für die digitale Signalübertragung zwischen der Bedieneinrichtung, dem Steuerrechner und den Geräten eignet.

Gemäß einer Variante der Erfindung ist das Gerätecenter mit Aufnahmevorrichtungen für die medizinisch-technischen Geräte versehen. Die Aufnahmevorrichtungen sind vorzugsweise gleichartig, können jedoch auch teilweise voneinander verschieden ausgeführt sein, so daß alle für einen offenen oder minimal invasiven chirurgischen Eingriff erforderlichen medizinisch-technische Geräte in das Gerätecenter integriert werden können. Dabei können unterschiedliche, aber in ihren Anschlüssen weitgehend identische Geräte unter Umständen von ein und derselben Aufnahmevorrichtung aufgenommen werden. Dem Gerätecenter können einem Baukastensystem vergleichbar Geräte sowohl hinzugefügt als auch entnommen werden. Auf diese Weise läßt sich der Systemarbeitsplatz mit unterschiedlichen Gerätekombinationen konfigurieren, welche der jeweiligen OP-Umgebung angepaßt werden können. Für die Instrumente der in Frage kommenden Geräte bzw. Steuerungen der Geräte sind entsprechende Anschlüsse an der Anschlußeinheit des Systemarbeitsplatzes vorgesehen. Bei den in Frage kommenden Geräten handelt es sich beispielsweise um HF-Generatoren, Saug/-Spülpumpen, Ultraschallgeräte, Insufflatoren, Endoskopkameras, Kaltlichtgeräte, Bohrgeräte, Laser etc. bzw. deren Steuerungen.

Eine Ausführungsform der Erfindung sieht vor, daß der Steuerrechner in das Gerätecenter integriert ist. Diese Ausführungsform der Erfindung ist besonders vorteilhaft, weil auf diese Weise kein weiterer Platzbedarf im Operationssaal für den Steuerrechner benötigt wird. Darüber hinaus entfallen die ansonsten notwendigen Verbindungsleitungen zwischen Gerätecenter und Steuerrechner, welche Hindernisse (Stolpergefahr) im Operationssaal darstellen würden.

Eine Variante der Erfindung sieht vor, daß das Gerätecenter mit einer Aufnahmevorrichtungen für mindestens einen Bildspeicher versehen ist. Die Bereitstellung eines Bildspeichers ermöglicht den Abruf gespeicherter Bildinformationen, beispielsweise während eines chirurgischen Eingriffes direkt durch den Chirurgen, so daß der Chirurg neu aufgenommene Bildinformationen mit bereits aufgezeichneten und gespeicherten Bildinformationen vergleichen kann, falls dies erforderlich ist. Der Vergleich der Bildinformationen kann sequentiell oder auch durch direkte Gegenüberstellung, beispielsweise in Form einer Doppelbilddarstellung auf zweigeteiltem Bildschirm, erfolgen.

Gemäß einer besonders bevorzugten Ausführungsform der Erfindung weist die Verbindungseinheit Verbindungsleitungen in Form von elektrischen Verbindungskabeln und/oder Lichtwellenleitern und/oder Druckluftleitungen und/oder Gasleitungen auf, wobei der Verbindungseinheit elektrische Verbindungskabel, Lichtwellenleiter, Druckluftleitungen oder Gasleitungen hinzugefügt oder entnommen werden können. Auf diese Weise werden die Instrumente der medizinisch-technischen Geräte mit elektrischer Energie, Druckluft oder Gas für ihren bestimmungsgemäßen Betrieb versorgt. Des weiteren erfolgt über die elektrischen Verbindungskabel oder Lichtwellenleiter beispielsweise die Übertragung von Bildsignalen, Licht oder die Übertragung von Steuersignalen. Die Vielfalt der zur Verfügung gestellten Verbindungsleitungen bzw. die Erweiterung um zusätzliche Verbindungsleitungen ermöglicht dabei den Einsatz der unterschiedlichsten medizinisch-technischen Geräte an dem Systemarbeitsplatz.

Gemäß einer weiteren Variante der Erfindung ist die Verbindungseinheit eine am Boden verlegte Flachbandleitung oder ein deckengehaltener Rillschlauch, wobei die Flachbandleitung oder der Rillschlauch die elektrischen Verbindungskabel, die Lichtwellenleiter, die Druckluftleitungen und die Gasleitungen aufnehmen, welche die Anschlußeinheit mit dem Gerätecenter und somit den einzelnen medizinisch-technischen Geräten verbinden. Auf diese Weise stellen die unterschiedlichen Verbindungsleitungen kein Hindernis im Operationssaal dar, da sie entweder in der Flachbandleitung integriert flach am Boden verlegt und leicht zu überqueren sind oder in den Rillschlauch integriert an der Decke außerhalb des Bewegungsraumes von Personen geführt sind.

Ein Ausführungsbeispiel der Erfindung ist in den beigefügten schematischen Zeichnungen dargestellt. Es zeigen:
- FIG 1: einen erfindungsgemäßen medizinisch-technischen Systemarbeitsplatz,
- FIG 2: in schematischer, geschnittener Darstellung die Flachbandleitung aus FIG 1,
- FIG 3: eine weitere Ausführungsform eines erfindungsgemäßen medizinisch-technischen Systemarbeitsplatzes, und
- FIG 4: in schematischer, geschnittener Darstellung den Rillschlauch aus FIG 3.

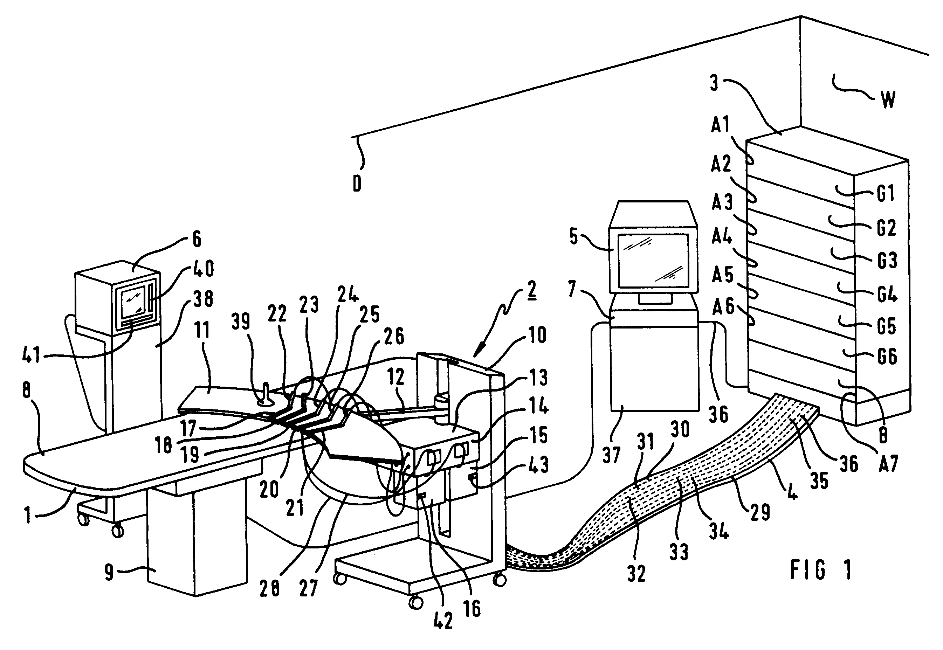

FIG 1 zeigt einen erfindungsgemäßen medizinisch-technischen Systemarbeitsplatz für die offene oder minimal invasive Chirurgie. Der Systemarbeitsplatz weist einen Patientenlagerungstisch 1, eine Darreichungseinheit 2, ein Gerätecenter in Form eines Geräteschrankes 3, eine Verbindungseinheit 4 und einen mit Monitoren 5 und 6 versehenen Steuerrechner 7 auf.

Der Patientenlagerungstisch 1 weist eine Patientenlagerungsplatte 8 und eine Hubsäule 9 auf, so daß die Patientenlagerungsplatte 8 in an sich bekannter Weise vertikal verstellbar ist.

An den Patientenlagerungstisch 1 ist die insgesamt mit 2 bezeichnete Darreichungseinheit herangefahren, die unter anderem einen auf Rädern verfahrbaren Gerätewagen 10 umfaßt. An dem Gerätewagen 10 ist eine bogenförmig ausgebildete wenigstens im wesentlichen horizontal ausgerichtete Ablageschale 11 angebracht, welche über einen höhenverstellbaren und schwenkbaren Tragarm 12 mit dem Gerätewagen 10 verbunden ist. Des weiteren weist die Darreichungseinheit 2 eine an dem Gerätewagen 10 angebrachte Anschlußeinheit 13 für Instrumente 22 - 26 medizinisch-technischer Geräte auf. Außerdem ist der Gerätewagen 10 mit einer im Falle des vorliegenden Ausführungsbeispiels im selben Gehäuse wie die Anschlußeinheit 13 angeordneten Pumpeinrichtung 14 und Gefäßen 15 und 16 für Spül- und Absaugflüssigkeiten versehen.

Die Ablageschale 11 ist mit Aufnahmevorrichtungen 17 - 21 für die als Handstücke ausgeführten Instrumente 22 - 26 versehen, in denen die Instrumente 22 - 26 für einen an dem Patientenlagerungstisch 1 arbeitenden Chirurgen in unmittelbarer Griffweite bereitgehalten werden. Die Aufnahmevorrichtungen 17 - 21 sind in FIG 1 in nicht näher dargestellten Aussparungen der Ablageschale 11 angeordnet und von der Ablageschale 11 abnehmbar. Die Aussparungen und die mit diesen zusammenwirkenden Bereiche der Aufnahmevorrichtungen sind derart gestaltet, daß verschiedene Aufnahmevorrichtungen in dieselbe Aussparung eingeführt werden können, d.h. Aufnahmevorrichtungen für verschiedene Instrumente 22 - 26 sind gegeneinander austauschbar. Die Abnehmbarkeit der Aufnahmevorrichtungen 17 - 21 erweist sich dabei für deren Sterilisierung als vorteilhaft. Die Ablageschale 11 kann durch die gegeneinander austauschbaren Aufnahmevorrichtung für unterschiedliche medizinische Eingriffe mit Aufnahmevorrichtungen für die jeweils erforderlichen Instrumenten konfiguriert werden, wobei, wie bereits erwähnt, die Instrumente in den dafür vorgesehenen an der Ablageschale 11 angeordneten entsprechenden Aufnahmevorrichtungen bereitgehalten werden. Dabei kann ein und dieselbe Aufnahmevorrichtung für die Aufnahme unterschiedlicher Instrumente geeignet sein.

Die von der Ablageschale 11 bereitgehaltenen Instrumente 22 - 26, bei denen es sich im Falle des vorliegenden Ausführungsbeispiels um eine Endoskopkamera mit integriertem Kaltlicht 22, ein Spül-/Sauginstrument 23, einen Ultraschallkopf 24, ein Insufflationsinstrument 25 und ein Bohrinstrument 26 handelt, sind mit ihren Anschlußleitungen über in FIG 1 nicht näher dargestellte lösbare Kupplungen an der Anschußeinheit 13 angeschlossen. Die Endoskopkamera mit integriertem Kaltlicht 22 ist dabei an einem elektrischen Anschluß und einem Lichtwellenleiteranschluß, das Spül-/Sauginstrument 23 mit seinen Schlauchleitungen 27, 28 an der Pumpeinrichtung 14, der Ultraschallkopf 24 an einem elektrischen Anschluß, das Insufflationsinstrument 25 an einem Gasanschluß und das Bohrinstrument 26 an einem Druckluftanschluß angeschlossen. Die Endoskopkamera mit integriertem Kaltlicht 22 kann aber auch an nur einem Anschluß angeschlossen sein, welcher sowohl elektrische Kontakte als auch Kontakte für Lichtwellenleiter aufweist. Die Anschlüsse der Anschlußeinheit 13 können also derart ausgeführt sein, daß ein Anschluß verschiedenartige Kontakte, z. B. elektrische und optische Kontakte, aufweist. Die Anschlußeinheit 13 weist eine Vielzahl gleichartiger und verschiedener Anschlüsse auf, so daß praktisch alle in der offenen oder minimal invasiven Chirurgie verwendeten Instrumente medizinisch-technischer Geräte an die Anschlußeinheit 13 angeschlossen werden können. Die Anschlußeinheit 13 weist dabei mehr Anschlüsse als die Ablageschale 11 Aufnahmevorrichtungen auf, so daß die Ablageschale 11 beliebig mit verschiedenen Instrumenten konfiguriert werden kann.

Die Anschlußeinheit 13 und der Tragarm 12 stellen im übrigen die Schnittstelle zwischen dem sterilen Teil und dem unsterilen Teil des Gerätewagens 10 dar. Der Tragarm 12 ist von dem Gerätewagen 10 und die Ablageschale 11 von dem Tragarm 12 abnehmbar, wobei sowohl der Tragarm 12 als auch die Ablageschale 11 derart ausgeführt sind, daß sie autoklavierbar sind. Die medizinischen Instrumente 22 - 26 sind mit ihren Anschlußleitungen ebenfalls sterilisierbar ausgeführt und jederzeit austauschbar. Wird also die Darreichungseinheit 2 für einen medizinischen Eingriff vorbereitet, so sind zuvor die Ablageschale 11, der Tragarm 12 und die Instrumente 22 - 26 mit ihren Anschlußleitungen sterilisiert worden.

Das Spül-/Sauginstrument 23 ist mit seiner Schlauchleitung 27 zum Spülen mit einer Spülflüssigkeit über die Pumpeinrichtung 14 mit dem Gefäß 15 und mit seiner zweiten Schlauchleitung 28 zum Absaugen von Körperflüssigkeiten über die Pumpeinrichtung 14 an das Gefäß 16 angeschlossen. Die Pumpeinrichtung 14 ist ihrerseits mit der Anschlußeinheit 13 elektrisch verbunden. Auf diese Weise kann je nach Betriebsmodus der Pumpeinrichtung 14 während eines chirurgischen Eingriffes mit dem Spül-/Sauginstrument 23 entweder der Operationsbereich gespült oder Körperflüssigkeit, z.B. Blut, aus dem Operationsbereich abgesaugt werden.

Der Gerätewagen 10 ist über die als Flachbandleitung 29 ausgeführte Verbindungseinheit 4 mit dem Geräteschrank 3 verbunden, in dem die zu den Instrumenten 22 - 26 gehörigen Geräte bzw. Gerätesteuerungen angeordnet sind. Der Geräteschrank 3 selbst ist zum einen räumlich getrennt von dem Gerätewagen 10 angeordnet und zum anderen in einer Mindestentfernung von 1,5 Meter von dem Patientenlagerungstisch 1 bzw. der Anschlußeinheit 13, beispielsweise an einer Wand W des Operationssaales, plaziert. Auf diese Weise sind die Geräte zum einen zentral an einer das Operationsteam nicht störenden Stelle im Operationssaal angeordnet, zum anderen besteht die Möglichkeit, daß eine einzige Bedienperson alle medizinisch-technischen Geräte überwacht und auf mögliche Anweisungen, beispielsweise des Chirurgen, entsprechend bedient bzw. über den Status der Geräte Auskunft gibt. Der Geräteschrank 3 muß sich nicht notwendigerweise im Operationssaal befinden, sondern kann gegebenenfalls auch außerhalb des Operationssaales angeordnet sein.

Der Geräteschrank 3 ist im Falle des vorliegenden Ausführungsbeispiels mit Aufnahmevorrichtungen A1 - A6 für ein Ultraschallgerät G1, eine Spül-/Saugpumpensteuerung G2, eine Bohrgerätsteuerung G3, einen Insufflator G4, einen Kameracontroller G5, ein Kaltlichtgerät G6 und einer Aufnahmevorrichtung A7 für einen Bildspeicher B versehen. Die Aufnahmevorrichtungen A1 - A7 der einzelnen medizinisch-technischen Geräte und Steuerungen G1 - G6 und des Bildspeichers B sind im Falle des vorliegenden Ausführungsbeispiels im wesentlichen gleichartig ausgeführt und jeweils mit einem Netzanschluß versehen. Die Aufnahmevorrichtung A4 für den Insufflator G4 weist zusätzlich einen Anschluß für eine mit einer Kohlendioxidflasche verbundenen Schlauchleitung auf. Die Netzanschlüsse und der Kohlendioxidanschluß des Insufflators G4 sind in FIG 1 nicht dargestellt.

Wie bereits erwähnt, ist der Geräteschrank 3 mit dem Gerätewagen 10 über die Flachbandleitung 29 verbunden, wobei sowohl der Geräteschrank 3 als auch der Gerätewagen 10 entsprechende, in FIG 1 nicht näher dargestellte Schnittstellen für den Anschluß der Flachbandleitung 29 aufweisen. Die Flachbandleitung 29 enthält im Falle des vorliegenden Ausführungsbeispiels unter anderem elektrische Verbindungskabel 30, 31, 32, einen Lichtwellenleiter 33, eine Gasleitung 34 und eine Druckluftleitung 35. Auf diese Weise wird beispielsweise das Insufflationsinstrument 25 über die Anschlußeinheit 13 und die Gasleitung 34 mit dem Insufflator G4 verbunden, so daß nach entsprechender Ansteuerung des Insufflators G4 Kohlendioxid über die Gasleitung 34 an die Anschlußeinheit 13 und von dieser an das Insufflationsinstrument 25 geführt wird. In entsprechender Weise werden der Ultraschallkopf 24 und die Pumpeinrichtung 14 über die elektrischen Verbindungskabel 30, 31 mit dem Ultraschallgerät G1 und der Saug/-Spülpumpensteuerung G2, die Endoskopkamera mit integriertem Kaltlicht 22 über das elektrische Verbindungskabel 32 und den Lichtwellenleiter 33 mit dem Kameracontroller G5 und dem Kaltlichtgerät G6 und das Bohrinstrument 26 über die Druckluftleitung 35 mit der Bohrgerätsteuerung G3 verbunden.

Die Flachbandleitung 29, welche, wie erwähnt, über entsprechende Schnittstellen an dem Geräteschrank 3 und dem Gerätewagen 10, dessen Schnittstelle in nicht dargestellter Weise mit der Anschlußeinheit 13 verbunden ist, angeschlossen ist, ist derart verformbar ausgeführt, daß bei Verstellungen des Gerätewagens 10 relativ zu dem Geräteschrank 3 die Flachbandleitung 29 entsprechend nachgeführt werden kann. Die interne Leitungsführung des Geräteschrankes 3 von der Schnittstelle zu den einzelnen Geräten G1 - G6 ist nicht dargestellt.

Die in dem Geräteschrank 3 angeordneten medizinisch-technischen Geräte bzw. Gerätesteuerungen G1 bis G6 und der Bildspeicher B sind an einen gemeinsamen Kommunikationsbus 36 angeschlossen, wobei in FIG 1 die einzelnen Anschlüsse des Kommunikationsbusses 36 mit den Geräten G1 - G6 und den Bildspeicher B nicht gezeigt sind. Der Kommunikationsbus 36 ist mit einem auf einem Schrank 37 angeordneten Steuerrechner 7 verbunden, welcher mit einer menügesteuerten Software derart betrieben wird, daß von dem Steuerrechner 7 aus alle medizinisch-technischen Geräte G1 - G6 und der Bildspeicher B angesteuert werden können. Der Steuerrechner 7 ist dabei in in FIG 1 nicht dargestellter Weise mit einer Tastatur und einer Bedieneinrichtung, z.B. einer Maus, versehen. Auf diese Weise besteht die Möglichkeit, daß eine einzige Bedienperson von dem mit dem Monitor 5 versehenen Steuerrechner 7 aus alle medizinisch-technischen Geräte überwacht und auf Anweisung, beispielsweise des Chirurgen, entsprechend bedient. Der an dem Steuerrechner 7 angeschlossene im Falle des vorliegenden Ausführungsbeispiels auf einem Wagen 38 plazierte zweite Monitor 6 ist im übrigen im Blickfeld des operierenden Chirurgen angeordnet. Auf diesem Monitor werden beispielsweise in an sich bekannter Weise die mittels der Endoskopkamera 22 gewonnen Bilder angezeigt.

Im Falle des vorliegenden Ausführungsbeispiels ist die Ablageschale 11 mit einer zentralen Bedieneinrichtung in Form eines Joysticks 39 versehen, welcher derart mittels einer temperaturbeständigen, flexiblen Kunststoffhülle geschützt ausgeführt ist, daß er mit der Ablageschale 11 autoklavierbar ist. Der Joystick 39 ist ebenfalls über die Anschlußeinheit 13 und den Kommunikationsbus 36, welcher über die Flachbandleitung 29 geführt ist, mit dem Steuerrechner 7 verbunden, wobei die Anschlußeinheit 13 entsprechende nicht dargestellten Anschlüsse zum Anschluß von Bedieneinrichtungen an den Kommunikationsbus 36 aufweist. Auf diese Weise kann bei Bedarf, beispielsweise ein operierender Chirurg, von der Ablageschale 11 aus alle medizinisch-technischen Geräte G1 - G6 und den Bildspeicher B selbst steuern. Der Steuerrechner 7 wird dabei mit der bereits erwähnten menügesteuerten Software betrieben, wobei an dem Monitor 6 neben den angezeigten für die Operation erforderlichen Bildern, z.B. Kamerabildern der Endoskopkamera 22, eine vertikale 40 und eine horizontale 41 Menüleiste angezeigt werden. Der Chirurg kann einzelne Menüpunkte der Menüleisten 40, 41 mit einem in den Bildschirm des Monitors 6 vom Steuerrechner 7 eingeblendeten, dem Joystick 39 zugeordneten Cursor anwählen und beispielsweise Statusinformationen von einzelnen Geräten G1 - G6 abrufen oder Steueranweisungen an Geräte bzw. Gerätesteuerungen G1 - G6 geben. Beispielsweise besteht die Möglichkeit, die in dem Geräteschrank 3 angeordnete Saug/-Spülpumpensteuerung G2 der Pumpeinrichtung 14 derart anzusteuern, daß die Pumpeinrichtung 14 Spülflüssigkeit aus dem Gefäß 15 durch die Schlauchleitung 27 und das Instrument 23 pumpt oder über das Instrument 23 und die Schlauchleitung 28 Körperflüssigkeit in das Gefäß 16 absaugt. In entsprechender Weise sind auch andere Geräte über die menügesteuerte Eingabe ansteuerbar.

Der Joystick 39 arbeitet im Falle des vorliegenden Ausführungsbeispiels in Form einer Stufenschaltung, wobei bei Betätigung des Joystick 39 in eine entsprechende Richtung, d.h. Auslösung eines elektrischen Signals, der Cursor zu dem in Betätigungsrichtung nächstliegenden Menüpunkt springt. Der Joystick 39 weist außerdem einen in FIG 1 nicht dargestellten Auslöseknopf auf, dessen Betätigung die einem Menüpunkt zugeordnete Aktion auslöst. Der Joystick 39 muß jedoch nicht notwendigerweise in Stufenschaltung, sondern kann in an sich bekannter Betriebsweise eines Joystick arbeiten.

Des weiteren können anstelle des Joystick 39 oder zusätzlich zu dem Joystick 39 andere Bedieneinrichtungen, z.B. Fußschalter, eine Empfangseinheit zur Voice-Control, handbetätigte Schalter oder sonstige Schalteinrichtungen vorgesehen sein, welche an entsprechende Anschlüsse des Anschlußeinheit 13 angeschlossen sind.

Den Gefäßen 15 und 16 sind im Falle des vorliegenden Ausführungsbeispiels elektrische Füllstandssensoren 42, 43 zugeordnet, welche über die Anschlußeinheit 13 und den Kommunikationsbus 36 mit dem Steuerrechner 7 verbunden sind, so daß die aktuellen Füllstände der Gefäße 15, 16 in das Monitorbild des Monitors 6 zur Information des Chirurgen eingeblendet werden können. Auf diese Weise ist der Chirurg jederzeit über den Füllstand der Gefäße 15 und 16 informiert und kann zu gegebener Zeit deren Austausch veranlassen.

Die Ablageschale 11 ist in in FIG 1 nicht dargestellter Weise wenigstens im wesentlichen horizontal verstellbar ausgeführt, d.h. die Ablageschale 11 kann auf einer nicht dargestellten Schiene laufend bogenförmig verstellt werden. Die Ablageschale 11 dient im übrigen nicht nur der Aufnahme medizinisch-technischer Instrumente, sondern auch der Aufnahme einfacher medizinischer Instrumente, z. B. Klammern oder konventionellen Skalpellen, welche auf den neben dem Joystick 39 und den Aufnahmevorrichtungen 17 - 21 zur Verfügung stehenden Flächen der Ablageschale 11 abgelegt werden können.

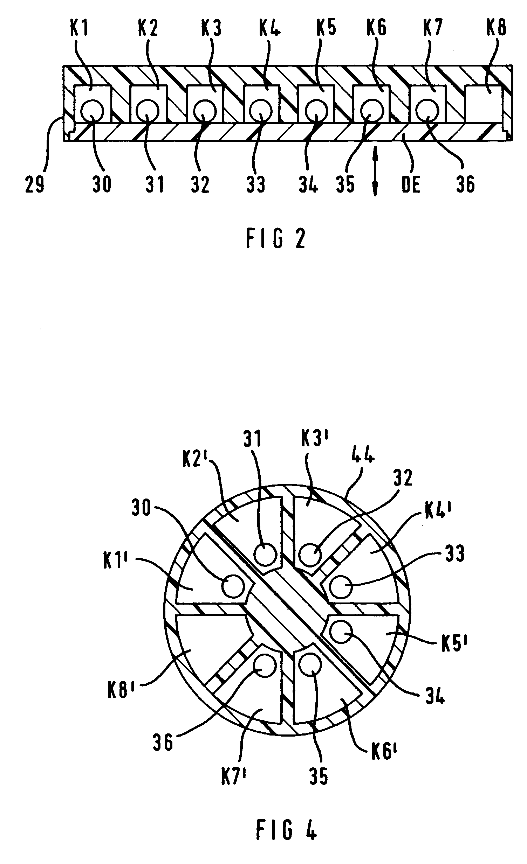

Die Flachbandleitung 29 ist entsprechend der baukastenartigen Ausführung des Geräteschrankes 3 derart ausgeführt, daß ihr elektrische Verbindungskabel, Lichtwellenleiter, Gasleitungen und Druckluftleitungen hinzugefügt bzw. entnommen werden können. FIG 2 zeigt exemplarisch in schematischer Weise den Aufbau der Flachbandleitung 29. Die Flachbandleitung 29 weist durch Stege voneinander getrennte Kammern K1 - K8 auf, wobei in den Kammern K1 - K7 die elektrischen Verbindungskabel 30, 31, 32, der Lichtwellenleiter 33, die Gasleitung 34, die Druckluftleitung 35 und der Kommunikationsbus 36 aufgenommen sind. Die Kammer K8 ist im Falle des vorliegenden Ausführungsbeispiels nicht belegt. Die Flachbandleitung 29 ist mit einem Deckel DE versehen, welcher die Kammern K1 - K8 verschließt. Der Deckel DE ist von der Flachbandleitung 29 abnehmbar (vgl. Doppelpfeil in FIG 2), so daß der Flachbandleitung 29 in nicht näher dargestellter Weise elektrische Verbindungskabel und/oder Lichtwellenleiter und/oder Gasleitungen und/oder Druckluftleitung hinzufügbar und entnehmbar sind. Die Flachbandleitung kann auch weniger oder mehr als die in FIG 2 dargestellten Kammern K1 - K8 aufweisen.

Die Flachbandleitung 29 muß im übrigen nicht notwendigerweise mit einer Schnittstelle des Gerätewagens 10, sondern kann auch direkt mit einer entsprechenden Schnittstelle der Anschlußeinheit 13 verbunden sein.

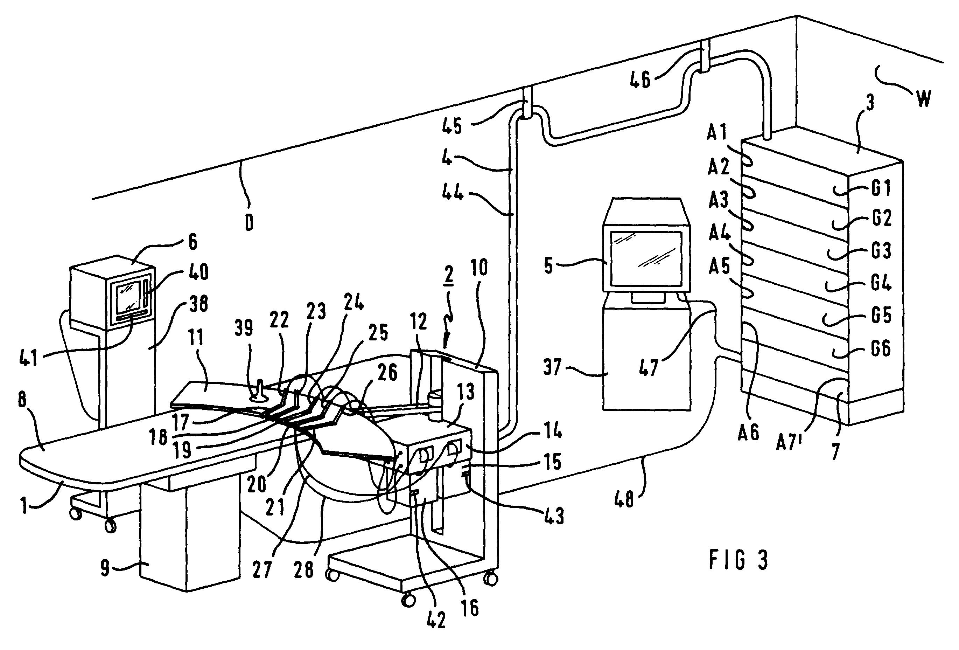

FIG 3 zeigt eine weitere Ausführungsform eines erfindungsgemäßen medizinisch-technischen Systemarbeitsplatzes, wobei Komponenten, welche mit Komponenten des Systemarbeitsplatzes aus FIG 1 zumindest weitgehend identisch sind, mit gleichen Bezugszeichen versehen sind. Der Unterschied des in FIG 3 dargestellten Systemarbeitsplatzes zu dem in FIG 1 dargestellten Systemarbeitsplatz besteht dabei darin, daß zum einen die Anschlußeinheit 13 des Gerätewagens 10 über einen Rillschlauch 44 mit dem Geräteschrank 3 verbunden ist.

Der in FIG 4 schematisch dargestellte Rillschlauch 44 weist, wie die Flachbandleitung 29 durch Stege voneinander getrennte Kammern K1' - K8' auf, wobei in den Kammern K1' - K7' die elektrischen Verbindungskabel 30, 31, 32, der Lichtwellenleiter 33 , die Gasleitung 34, die Druckluftleitung 35 und der Kommunikationsbus 36 aufgenommen sind. Die Kammer K8' ist im Falle des vorliegenden Ausführungsbeispiels nicht belegt. Dem Rillschlauch 44 können wie der Flachbandleitung 29 in nicht näher dargestellter Weise elektrischen Verbindungskabel und/oder Lichtwellenleiter und/oder Gasleitungen und/oder Druckluftleitungen entnommen bzw. hinzugefügt werden. Nicht mehr benötigte Leitungen werden dabei aus der sie aufnehmenden Kammer des Rillschlauchs 44 herausgezogen bzw. neue Leitungen in eine freie Kammer des Rillschlauchs 44 geschoben. Der Rillschlauch 44 kann auch weniger oder mehr als die in FIG 4 gezeigten Kammern K1' - K8' aufweisen.

Der Rillschlauch 44 ist durch an der Decke D des Operationssaales angeordnete Rillschlauchhalter 45, 46 geführt und weist eine derartige Länge auf, daß der Gerätewagen 10 mit dem an der Anschlußeinheit 13 angeschlossenen Rillschlauch 44 im Operationssaal praktisch frei verfahren werden kann. Die Rillschlauchhalter 45 und 46 können dabei in nicht dargestellter Weise in an der Decke D montierten Schienen bewegt werden, so daß sie in eine derartige Position gebracht werden können, daß der Rillschlauch 44 annähernd geradlinig vertikal von dem Rillschlauchhalter 45 zu dem Gerätewagen 10 verläuft. Auf diese Weise wird vermieden, daß der Rillschlauch 44 störend quer durch den Operationssaal verläuft. Die Anschlußeinheit 13 und der Geräteschrank 3 weisen in FIG 3 nicht dargestellte Schnittstellen zum Anschluß des Rillschlauches 44 auf.

Ein weiterer Unterschied des Ausführungsbeispieles gemäß FIG 3 gegenüber dem Ausführungsbeispiel gemäß FIG 1 besteht darin, daß der Steuerrechner 7 in den Geräteschrank 3 integriert ist. Der Steuerrechner 7 ist in diesem Fall in der Aufnahmevorrichtung A7 angeordnet. Der Kommunikationsbus 36 verbindet dabei in nicht dargestellter Weise wie im zuvor beschriebenen Ausführungsbeispiel die medizinisch-technischen Geräte G1 - G6, die Füllstandssensoren 42, 43, den Joystick 39 und den Steuerrechner 7 miteinander. An den Steuerrechner 7 sind wie im zuvor beschriebenen Ausführungsbeispiel über Monitorkabel 47 und 48 die Monitoren 5 und 6 angeschlossen.

Die Monitore 5 und 6 können im übrigen in nicht dargestellter Weise auf decken-, wand- oder bodenmontierten Gelenkarmen angeordnet sein, so daß sie entsprechend innerhalb des Operationssaales in das Blickfeld, beispielsweise des Chirurgen, positioniert werden können.

Darüber hinaus muß die Darreichungseinheit 2 nicht notwendigerweise einen Gerätewagen 10 aufweisen. Die Darreichungseinheit 2 kann vielmehr eine Haltevorrichtung sein, welche mittels eines decken-, wand- oder bodenmontierten Gelenkarmes gehalten wird und entsprechend im Operationssaal verstellt werden kann.

Des weiteren müssen die Ablageschale 11 und die Anschlußeinheit 13 nicht notwendigerweise an der Darreichungseinheit 2 angeordnet sein. Vielmehr können die Ablageschale 11 und die Anschlußeinheit an eigenen, von einander unabhängig bewegbaren Halterungen angeordnet sein.

Die Ausführungsform des Gerätecenters als Geräteschrankes 3 ist nur exemplarisch zu verstehen. Der Geräteschrank 3 kann dabei in nicht dargestellter Weise auch über mehr als die angegebenen Aufnahmevorrichtungen für medizinisch-technische Geräte, beispielsweise einen Laser, und weitere Bildspeicher verfügen. Sind dabei nicht alle medizinisch-technischen Geräte in einen Geräteschrank integrierbar, so besteht die Möglichkeit, einen zweiten Geräteschrank hinzunehmen, welcher entsprechend mit der Anschlußeinheit 13 verbunden ist.

Im übrigen kann der erfindungsgemäße Systemarbeitsplatz auch mehrere Steuerrechner, mehrere Verbindungseinheiten und die Darreichungseinheit mehrere Anschlußeinheiten aufweisen.

Der in der Flachbandleitung 29 bzw. dem Rillschlauch 44 geführte Kommunikationsbus 36 ist im übrigen als elektrisches Verbindungskabel zu betrachten, welches der Flachbandleitung 29 bzw. dem Rillschlauch 44 entnommen und hinzugefügt werden kann.

## Patentansprüche

1. Medizinisch-technischer Systemarbeitsplatz für die offene oder minimal invasive Chirurgie aufweisend eine Ablageschale (11) und mindestens eine Anschlußeinheit (13) für Instrumente (22 - 26) medizinisch-technischer Geräte (G1 - G6), mindestens ein räumlich von der Anschlußeinheit (13) getrenntes Gerätecenter (3) zur Aufnahme der medizinisch-technischen Geräte (G1 - G6) und mindestens eine Verbindungseinheit (4), welche die Anschlußeinheit (13) und das Gerätecenter (3) miteinander verbindet.

2. Medizinisch-technischer Systemarbeitsplatz nach Anspruch 1, bei dem die Ablageschale (11) und die Anschlußeinheit (13) an einer Darreichungseinheit (2) angeordnet sind.

3. Medizinisch-technischer Systemarbeitsplatz nach einem der Ansprüche 1 oder 2, bei dem die Ablageschale (11) mindestens eine Bedieneinrichtung (39) aufweist, welche über die Verbindungseinheit (4) mit mindestens einem Steuerrechner (7) des Systemarbeitsplatzes verbunden ist, mit welchem die medizinisch-technischen Geräte (G1 - G6) verbunden sind.

4. Medizinisch-technischer Systemarbeitsplatz nach Anspruch 3, bei dem die medizinisch-technischen Geräte (G1 - G6), die Bedieneinrichtung (39) und der Steuerrechner (7) über einen Kommunikationsbus (36) miteinander verbunden sind.

5. Medizinisch-technischer Systemarbeitsplatz nach einem der Ansprüche 1 bis 4, bei dem das Gerätecenter (3) mit Aufnahmevorrichtungen (A1 - A6) für die medizinisch-technischen Geräte (G1 - G6) versehen ist.

6. Medizinisch-technischer Systemarbeitsplatz nach einem der Ansprüche 3 bis 5, bei dem der Steuerrechner (7) in das Gerätecenter (3) integriert ist.

7. Medizinisch-technischer Systemarbeitsplatz nach einem der Ansprüche 1 bis 6, bei dem das Gerätecenter (3) mit einer Aufnahmevorrichtung (A7) für mindestens einen Bildspeicher (B) versehen ist.

8. Medizinisch-technischer Systemarbeitsplatz nach einem der Ansprüche 1 bis 7, bei dem die Verbindungseinheit (4) Verbindungsleitungen in Form von elektrischen Verbindungskabeln (30, 31, 32, 36) und/oder Lichtwellenleitern (33) und/oder Druckluftleitungen (35) und/oder Gasleitungen (34) aufweist.

9. Medizinisch-technischer Systemarbeitsplatz nach Anspruch 8, bei dem der Verbindungseinheit (4) elektrische Verbindungskabel (30, 31, 32, 36) und/oder Lichtwellenleiter (33) und/oder Druckluftleitungen (35) und/oder Gasleitungen (34) hinzufügbar und entnehmbar sind.

10. Medizinisch-technischer Systemarbeitsplatz nach Anspruch 8 oder 9, bei dem die Verbindungseinheit (4) eine am Boden verlegte Flachbandleitung (29) ist.

11. Medizinisch-technischer Systemarbeitsplatz nach Anspruch 8 oder 9, bei dem die Verbindungseinheit (4) ein deckengehaltener Rillschlauch (44) ist.
